# EUROPÄISCHE PATENTSCHRIFT

(11) **EP 3 681 598 B1**
(45) Veröffentlichungstag und Bekanntmachung des Hinweises auf die Patenterteilung: **15.11.2023**
(21) Anmeldenummer: 18765634.3
(22) Anmeldetag: 06.09.2018
(51) Int. Cl.: A61N 5/06

(54) **IMPLANTATSYSTEM**
IMPLANT SYSTEM
SYSTÈME D'IMPLANT

(30) Priorität: 11.09.2017 DE 102017120949
(43) Veröffentlichungstag der Anmeldung: 22.07.2020
(73) Patentinhaber: Halatsch, Marc-Eric, 89081 Ulm (DE); Capanni, Felix, 89233 Neu-Ulm (DE)
(72) Erfinder: HALATSCH, Marc-Eric, 89081 Ulm (DE); CAPANNI, Felix, 89233 Neu-Ulm (DE); KARPEL-MASSLER, Georg, 70182 Stuttgart (DE); KAST, Richard, Eric, Burlington VT 05408 (US)
(74) Vertreter: Hentrich Patent- & Rechtsanwaltspartnerschaft mbB
(86) Internationale Anmeldenummer: PCT/EP2018/074039
(87) Internationale Veröffentlichungsnummer: WO 2019/048559

(56) Entgegenhaltungen:
- EP-A1- 1 334 748
- CN-U- 202 070 026
- DE-A1- 19 819 975
- DE-A1-102014 107 298
- RU-C1- 2 346 712
- US-A1- 2002 087 206
- US-A1- 2006 111 762
- US-A1- 2010 305 666
- US-A1- 2014 128 943

## Beschreibung

Die Erfindung betrifft ein Implantatsystem für die photodynamische Therapie mit einem in eine Resektionshöhle implantierbaren Leuchtkörper zur Abstrahlung von Licht und mit einer autonomen Steuereinheit, die über eine Versorgungsleitung mit dem Leuchtkörper verbindbar ist und ein Reservoir für mindestens ein Medikament aufweist, wobei das Reservoir über eine Medikamentenleitung mit dem Leuchtkörper verbunden ist, der zur Abgabe des mindestens einen Medikamentes an das umliegende Gewebe eingerichtet ist, wobei die Medikamentenleitung parallel zur Versorgungsleitung verläuft, und wobei der Leuchtkörper Poren und/oder auf seiner Oberfläche Kanäle zur Verteilung der Medikamente aufweist.

Glioblastome sind die am weitesten verbreiteten und aggressivsten Gehirntumore bei erwachsenen Menschen. Eine Erkrankung mit einem derartigen bösartigen Hirntumor ist bis heute nicht heilbar und dem erkrankten Menschen verbleibt nur eine mittlere Überlebenszeit von ca. 16 Monaten. Ein Therapieansatz ist dabei, den bösartigen Hirntumor operativ zu entfernen in der Kombination mit einer intraoperativen photodynamischen Therapie, bei der dem Patienten ein Medikament zugeführt wird, das sich möglichst selektiv in den Tumorzellen anreichert und bei Bestrahlung mit Licht geeigneter Wellenlänge phototoxisch wirkt. Die Entfernung des bösartigen Tumors, dessen Positionierung im Hirn eine vorsorgliche weiträumige Entfernung auch gesunden Gewebes verhindert, hinterlässt eine Resektionshöhle mit einer Resektionswand, an der häufig bösartige Zellen verbleiben. Es besteht die Möglichkeit, dass mittels einer durch die offene Operationswunde in die Resektionshöhle eingeführten Lampe das von den Tumorzellen aufgenommene Medikament aktiviert wird, um an der Resektionswand verbliebene bösartige Zellen zu zerstören.

Nachteilig dabei ist, dass diese Therapiemöglichkeit nur während der Operation an der offenen Operationswunde besteht und die Zeitdauer der photodynamischen Therapie zur Vermeidung einer zu starken Belastung des Patienten nicht beliebig ausgedehnt werden kann.

Die US 2002/0087 206 A1 betrifft die Anwendung der photodynamischen Therapie unter Verwendung eines aufblasbaren Ballons zur Stabilisierung der Wände einer Resektionshöhle, wobei des Weiteren eine lichtstreuende Flüssigkeit in den aufblasbaren Ballon eingeführt wird, in die über eine optische Faser Licht eingebracht und von dort verteilt werden kann.

In der US 2010/0305666 A1 wird für die photodynamische Therapie vorgeschlagen, in einem Implantat eine Lichtquelle bereitzustellen, die durch Induktion mit Energie versorgt wird, wie insbesondere in dem Absatz [0016] zu erläutert ist:
In der US 2014/0128943 A1 wird für die photodynamische Therapie vorgeschlagen, in einer Körperkavität eine Lichtquelle zu implantieren, die über einen Lichtleiter versorgt wird.

Die EP 1 334 748 A1 offenbart die Merkmale des Oberbegriffs von Anspruch 1 und betrifft gleichfalls zur Anwendung in der photodynamischen Therapie eine Vorrichtung, bei der am Ende eines Lichtstabs, eine bzw. eine Mehrzahl von LED angeordnet ist, wobei dieses Implantat in dem Körper des Patienten während eines chirurgischen Eingriffes verwendet wird und auch dort für einige Tage oder einen längeren Zeitraum verbleiben kann.

Zusätzlich ist offenbart, dass von einem externen Reservoir durch einen Katheter ein photoreaktiver Stoff der Behandlungsstelle zugeführt werden kann.

Die DE 10 2014 107 298 A1 betrifft eine optische Stimulationseinrichtung, die zum Implantieren in einem von einer Körperflüssigkeit durchflossenen natürlichen inneren Hohlraum des Körpers eines Lebewesens eingerichtet ist, bei der die Implantat-Komponente eine Trägerstruktur besitzt, an der eine Vielzahl von Elektroden befestigt sind und in der mehrere Öffnungen und/oder Kanäle ausgebildet sind, durch die nach der Implantation am Körper die Körperflüssigkeit strömen kann.

Die CN 202070026U betrifft die Anwendung der photodynamischen Therapie, wobei zur Befestigung ist eine Fahne vorgesehen.

Die RU2346712 C1 offenbart die prinzipielle Anwendung der photodynamischen Therapie ebenso wie dieUS 2006/0111762 A1.

Die DE 198 19 975 A1 verweist allgemein auf den Einsatz photodynamischer Therapien, wobei die Nutzung reflektierender Schablonen zum Schutz umgebender Bereiche offenbart ist. Der Erfindung liegt daher die Aufgabe zugrunde, zur Erhöhung der mittleren Überlebenszeit von tumorerkrankten Patienten die Anwendbarkeit der photodynamischen Therapie zu verbessern.

Diese Aufgabe wird durch das eingangs genannte Implantatsystem gelöst, mit dem die Möglichkeit geschaffen ist, in die durch die Entfernung des Tumors entstandene Resektionshöhle einen Leuchtkörper zu implantieren, durch den Licht mit therapeutisch wirksamer Wellenlänge abgestrahlt werden kann, so dass durch Lichtexposition von Tumorzellen, die mit einem phototoxischen Medikament angereichert sind, auch nach der Operation und dem Verschließen der Operationswunde die photodynamische Therapie fortgesetzt werden kann. Eine verbesserte Möglichkeit zur Bekämpfung von Tumoren beziehungsweise Tumorzellen ergibt sich, da die Steuereinheit ein Reservoir für ein Medikament aufweist, wobei das Reservoir über eine Medikamentenleitung mit dem Leuchtkörper verbunden ist, der zur Abgabe des Medikamentes an das umliegende Gewebe eingerichtet ist. Das bereitgestellte Medikament kann dabei dasselbe wie für die Phototoxizität verwendete sein oder ein ergänzendes Medikament.

Da prinzipiell jede Resektionshöhle geeignet ist, ist die Anwendung des Implantatsystems nicht auf Glioblastome beschränkt, sondern kann auch für die photodynamische Therapie von beispielsweise Leber-, Pankreas-, Nieren-, Harnblasen-, Prostata-, Brochialkarzinomen, Kopf-Hals-Tumoren auf HNO-Gebiet, Mamma-Karzinomen nach brusterhaltender OP mit hohem Rezidivrisiko, Endometrium- (Zervix-Karzinomen nach uteruserhaltender Resektion bei Kinderwunsch mit hohem Rezidivrisiko u. a. ausgedehnt werden.

Die Überlebungschance des an einem Glioblastom erkrankten Patienten ist erhöht, wenn eine frühzeitige Entfernung möglich ist, also bevor das Glioblastom stark gewachsen ist und die damit einhergehende Raumforderung nach der Entfernung des Glioblastoms eine große Resektionshöhle zurücklässt. Es liegt daher in der Regel eine relativ kleine Resektionshöhle vor. Um den Leuchtkörper lange betreiben zu können, ist die autonome Steuereinheit vorgesehen, die räumlich getrennt von dem Leuchtkörper auch außerhalb des Schädels angeordnet werden kann, wobei der Abstand zwischen dem Leuchtkörper und der Steuereinheit über die Länge der Versorgungsleitung bemessen werden kann. Sowohl die Versorgungsleitung als auch die autonome Steuereinheit können subkutan, intrakorporal angeordnet und damit zur Komplettierung des Implantatsystems dauerhaft implantiert werden.

Durch die räumliche Trennung der autonomen Steuereinheit von dem Leuchtkörper kann unabhängig von der Größe der Resektionshöhle ein angemessenes Volumen in der autonomen Steuereinheit für die Energieversorgung und Steuerung der Betriebsweise des Leuchtkörpers bereitgestellt werden.

Im Rahmen der Erfindung ist weiterhin vorgesehen, dass der Leuchtkörper aus einem lichtdurchlässigen Material, insbesondere transparentem Kunststoff oder Glas ausgebildet ist und dass gegebenenfalls im lichtdurchlässigen Material Streuzentren zur Förderung einer gleichmäßigen Ausleuchtung ausgebildet sind. Die Verwendung von Glas für optische Zwecke hat sich bewährt, insbesondere kann durch eine geringe Absorption sichergestellt werden, dass eine ausreichende Lichtstärke zur Aktivierung des phototoxischen Medikaments zur Verfügung steht, auch wenn zur Erreichung einer möglichst langen Leuchtdauer des Implantats der Energieverbrauch auf eine möglichst geringe Abstrahlung von Licht optimiert ist. Eine Bruchgefahr ist wegen der Platzierung des Leuchtmittels im Hirn und damit innerhalb der Schädelkalotte nicht gegeben.

Eine weitere Möglichkeit zur Kontrolle der Lichtabstrahlung ergibt sich insbesondere, wenn auf dem Leuchtkörper zur partiellen Ausleuchtung eine Maskierung vorgesehen ist.

Im Rahmen der Erfindung besonders bevorzugt ist es, wenn der Leuchtkörper aus zwei Halbschalen gebildet ist, zwischen denen mindestens ein Leuchtmittel aufgenommen ist, das mittels der Versorgungsleitung mit der in der autonomen Steuereinheit angeordneten Spannungsquelle verbindbar ist.

Im Rahmen der Erfindung besteht auch die Möglichkeit, dass das Leuchtmittel mehrfach vorgesehen ist und dass jedes der Leuchtmittel durch eine LED gebildet ist. LEDs zeichnen sich durch ihre lange Lebensdauer und ihren geringen Energieverbrauch aus, wobei in der Regel aber ein relativ geringer Abstrahlungswinkel gegeben ist. Dies kann zur Erreichung der bevorzugten sphärischen Abstrahlung durch die Nutzung mehrerer LEDs kompensiert werden, wobei die Anordnung der LEDs für eine gleichmäßige sphärische Ausleuchtung gewählt ist. Dazu sind die LEDs auf einem LED-Träger angeordnet, der als Anschlussglied der LEDs mit der Versorgungsleitung ausgebildet ist. Als günstig hat es sich dabei erwiesen, wenn der LED-Träger die Form einer Kugel, oder die Form eines Quaders, einschließlich der Sonderform eines Würfels, oder die Form einer Pyramide aufweist, auf deren Flächen im Flächenschwerpunkt die LEDs angeordnet sind.

Im Rahmen der Erfindung weiterhin besonders bevorzugt ist es, wenn zwei Koppelglieder zur lösbaren Verbindung der Versorgungsleitung mit der Steuereinheit vorgesehen sind, von denen eines an der Außenwandung des Gehäuses der Steuereinheit angeordnet ist. Dies bietet Vorteile während der Operation bei der Implantation des Implantatsystems, da die Möglichkeit besteht, den Leuchtkörper in der durch die Entfernung des Tumors entstandenen Resektionshöhle zu platzieren und sodann die Versorgungsleitung subkutan in den Oberkörper des Patienten zu verlegen. Dort wird die Verbindungsleitung mit dem ihr zugeordneten Koppelglied an das an der Außenwandung des Gehäuses der Steuereinheit angeordnete Koppelglied gekoppelt.

Weiterhin ist die Möglichkeit geschaffen, dass mehrere Leuchtkörper unterschiedlichen Volumens vorgesehen sind zur wahlweisen Verbindung eines der Leuchtkörper durch die zugeordnete Versorgungsleitung mit dem Koppelglied der Steuereinheit. Es ist damit ein modular aufgebautes Implantatsystem bereitgestellt, bei dem die Größe des Leuchtkörpers möglichst genau an die Größe der Resektionshöhle angepasst werden kann, um die direkte Abstrahlung des Lichtes an die dem vormaligen Tumor zugewandte Resektionsgrenzschicht des Gehirns zu ermöglichen, ohne dass durch Heilungsprozesse sich Ablagerungen/Narben zwischen der Oberfläche des Leuchtkörpers und der Grenzschicht ausbilden können.

Im Rahmen der Erfindung ist auch die Möglichkeit geschaffen, dass mehrere Leuchtkörper mit Leuchtmitteln unterschiedlicher Abstrahlfrequenzvorgesehen sind zur wahlweisen Verbindung eines der Leuchtkörper durch die zugeordnete Versorgungsleitung mit dem Koppelglied der Steuereinheit. Dies schafft die Möglichkeit, dass zur Erzielung eines maximalen phototoxischen Effektes die dafür geeigneten Wellenlängen zur Aktivierung variierbarer Medikamente zur Verfügung stehen. Es kann auch ein im UV-Bereich bei ca. 280 nm abstrahlendes Leuchtmittel genutzt werden in Kombination mit Leuchtmitteln zur phototoxischen Aktivierung. Das Leuchtmittel im UV-Bereich bewirkt eine tumorizidale Wirkung am Tumorresektionsrand auch ohne phototoxisches Medikament.

Im Rahmen der Erfindung hat es sich weiterhin als vorteilhaft erwiesen, wenn die Steuereinheit über mindestens eine wiederaufladbare elektrische Speicherzelle zur Spannungsversorgung der mindestens einen LED sowie einer Steuerplatine verfügt, wobei die Speicherzelle vorzugsweise durch einen Akkumulator gebildet ist, der induktiv geladen werden kann, also zur Verlängerung der Nutzungsdauer des Implantatsystems im implantierten Zustand eine Aufladung der Speicherzelle ohne erneuten operativen Eingriff möglich ist. Denkbar ist auch ein Datenaustausch zwischen der implantierten Steuereinheit und einer extrakorporalen Sende- und Empfangseinheit, um beispielsweise Daten über die Behandlung oder den Energieverbrauch aus einem der Steuereinheit zugeordneten Speicher auszulesen oder das Programm der Steuereinheit anzupassen. Der Datenaustausch erfolgt dabei kabellos durch Funk, beispielsweise NFC oder RFID.

Der Leuchtkörper ist innerhalb der Schädelkalotte relativ gut geschützt, während die Steuereinheit in der Regel subkutan positioniert und damit gegenüber äußeren Einflüssen exponiert ist. Es ist daher vorgesehen, dass das Gehäuse der Steuereinheit aus Implantatstahl oder Titan oder einem biokompatiblen Kunststoff, beispielsweise PEEK gebildet ist. Die Ausbildung des Gehäuses aus Kunststoff oder mit einem Kunststofffenster begünstigt den Datenaustausch per Funk und die induktive Aufladung der Speicherzelle in der Steuereinheit. Außerdem werden bei bildgebenden Verfahren weniger Artefakte verursacht. Günstig ist es weiterhin, wenn der Leuchtkörper und/oder die Versorgungsleitung und/oder die Steuereinheit und/oder die Medikamentenleitung mit einem biokompatiblen Material ummantelt sind, das sich von dem biokompatiblen Kunststoff durchaus unterscheiden kann. Als geeignet hat sich für die Ummantelung erwiesen, wenn das biokompatible Material durch medizinisches Silikon gebildet ist.

Mit einem derartigen Implantatsystem besteht damit die Möglichkeit, dass über einen langen Zeitraum eine photodynamische Therapie durchgeführt werden kann, insbesondere weil durch die von den Leuchtkörper räumlich getrennte Steuereinheit ein ausreichend großer Energiespeicher, gegebenenfalls wieder aufladbar, bereitgestellt ist und weiterhin in der Steuereinheit ausreichend Platz zur Verfügung steht, um durch Microcontroller auf der Steuerplatine den Betrieb des Leuchtkörpers zu steuern. Dabei besteht die Möglichkeit, dass kontinuierlich der Leuchtkörper Licht abstrahlt, oder das die Abstrahlung des Lichtes getaktet erfolgt, oder dass die Intensität des abgestrahlten Lichtes zeitlich variiert wird. Sofern mehrere LEDs als Leuchtmittel eingesetzt werden, besteht auch die Möglichkeit, dass diese LEDs bei unterschiedlichen Wellenlängen abstrahlen und dass durch den Microcontroller die Abstrahldauer und Intensität jeder einzelnen LED gesteuert wird.

Ganz besonders bevorzugt ist eine Ausführungsform, bei der im Inneren des Leuchtkörpers mindestens ein Photosensor angeordnet ist, dessen Signal über eine der Versorgungsleitung zugeordnete Signalleitung zu einer in der Steuereinheit angeordnete Auswerteeinheit zum Schalten des Leuchtkörpers zuführbar ist. Die Ausführungsform ermöglicht eine sehr vorteilhafte Betriebsweise des Implantatsystems, die ebenfalls Gegenstand der vorliegenden Offenbarung ist. Hierdurch ist nunmehr ein theragnostisches Implantat bereitgestellt, bei dem die Möglichkeit besteht, durch Gabe eines sich im Tumor anreichernden, Fluoreszenz hervorrufenden Wirkstoffes mittels des Leuchtkörpers Licht geeigneter Wellenlänge abzustrahlen und mittels eines an die zu detektierende Wellenlänge angepassten Filters und Photodetektors das Auftreten von Fluoreszenz zu überprüfen. Ist diese durch den Photodetektor, welcher gebildet ist durch den Photosensor und die räumlich getrennt angeordnete Auswerteeinheit, feststellbar, ist dies ein diagnostischer Hinweis auf die Präsenz von Tumorzellen, der die therapeutische Verwendung des Implantatsystems durch Einschalten des Leuchtkörpers indiziert, der zur Therapie das phototoxische Medikament aktiviert. Bei Verwendung mehrerer Leuchtmittel innerhalb des Leuchtkörpers kann ein Einschalten auch nur für die den Tumorzellen zugewandten Leuchtmittel erfolgen, um den Energieverbrauch zu optimieren.

Für eine langandauernde Therapie ist hier eine getaktete Betriebsweise vorteilhaft, bei der in durch den Arzt gewählten und in der Steuereinheit hinterlegten Abständen, synchronisiert mit der Medikamentengabe, der Leuchtkörper zur Suche nach Fluoreszenz aktiviert wird, um bei Detektion von Fluoreszenz in den Therapiemodus zu wechseln zur Erzeugung der phototoxischen Wirkung des dazu vorgesehenen Medikaments.

Ein zur Erzeugung von Fluoreszenz geeignetes Medikament ist beispielsweise 5-ALA (Aminolävulinsäure). Als photosensitive, therapeutisch wirksame Substanzen sind beispielsweise Porphyrine, Cyanine, Metatetrahydrophenylchlorine und andere geeignet.

Die Verwendung eines Leuchtkörpers aus Glas ermöglicht auch die diagnostische Anwendung bildgebender Verfahren zur Verlaufskontrolle einschließlich MRI. Auch besteht die Möglichkeit, dass dem Leuchtkörper eine auf das Tumorgebiet gerichtete Kamera zugeordnet ist, deren Daten über eine zur Versorgungsleitung parallele Kameraleitung an die Steuereinheit 3 übermittelbar und/oder dort speicherbar und/oder kabellos an einen extrakorporalen Empfänger übertragbar sind oder bei entsprechender Indikation, das Implantatsystem wieder zu entfernen und den Leuchtkörper aus der Resektionshöhle zu entnehmen und eine erneute Resektion durchzuführen oder den Leuchtkörper auszutauschen. Im folgenden wird die Erfindung an in der Zeichnung dargestellten Ausführungsbeispielen näher erläutert; es zeigen:
- Fig. 1: eine schematische Darstellung des aus Leuchtkörper, Versorgungsleitung und Steuereinheit gebildeten Implantatsystems,
- Fig. 2: eine schematische Darstellung des an einem Menschen positionierten Implantatsystems,
- Fig. 3: eine perspektivische, isolierte Darstellung eines mehrere Leuchtmittel aufweisenden Leuchtmittel-Trägers in Quaderform,
- Fig. 4: eine der Figur 3 entsprechende Darstellung mit dem Träger in Form einer Pyramide,
- Fig. 5: eine schematische Darstellung, einseitig geöffnet, der Steuereinheit mit einer Mehrzahl an Speicherzellen und einer einen Microcontroller aufweisenden Steuerplatine, und
- Fig. 6: eine der Figur 3 entsprechende Darstellung einer Ausführungsform mit einem Photosensor.

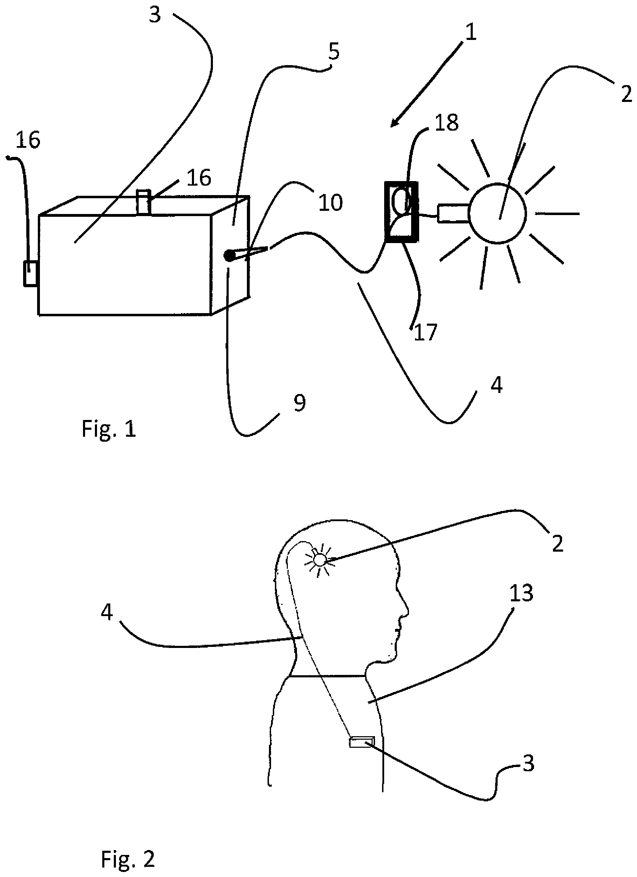

In Figur 1 ist ein Implantatsystem 1 gezeigt, das für den Einsatz in der photodynamischen Therapie bestimmt ist. Dieses Implantatsystem 1 umfasst einen in eine Resektionshöhle implantierbaren Leuchtkörper 2 zur Abstrahlung von Licht, eine autonome Steuereinheit 3 sowie eine Versorgungsleitung 4 zur Verbindung des Leuchtkörpers 2 mit der autonomen Steuereinheit 3.

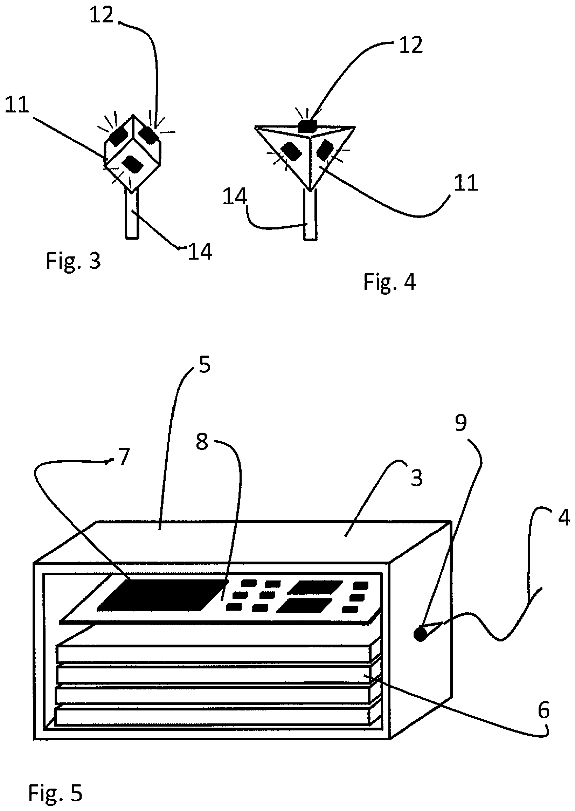

Die in der Figur 5 dargestellte autonome Steuereinheit 3 verfügt über ein Gehäuse 5, in dem mindestens eine elektrische Speicherzelle 6 angeordnet ist, wobei bei dem gezeigten Ausführungsbeispiel insgesamt 4 als Akkumulatoren gebildete elektrische Speicherzellen 6 vorhanden sind. In dem Gehäuse 5 weiterhin angeordnet ist eine einen Microcontroller 7 aufweisende Steuerplatine 8. Erkennbar ist auch, dass durch die Wandung des Gehäuses 5 ein Koppelglied 9 geführt ist, an dem als Gegenstück ein der Versorgungsleitung 4 zugeordnetes Koppelglied 10 lösbar befestigt werden kann. Diese Versorgungsleitung 4 ist zu einem LED-Träger 11 geführt, dessen Gestaltung so gewählt ist, dass eine gleichmäßige sphärische Ausleuchtung durch eine Mehrzahl von LEDs 12 möglich ist, die das Leuchtmittel bilden. Die Verwendung anderer Leuchtmittel ist gleichfalls denkbar. Gezeigt in Figur 3 ist der LED-Träger 11 in der Form eines Quaders, nämlich eines Würfels, während Figur 4 den LED-Träger 11 in der Form einer Pyramide zeigt. Der LED-Träger 11 fungiert als Anschlussglied der LEDs 12 zu der Versorgungsleitung 4.

Der in Figur 1 symbolisiert dargestellte Leuchtkörper 2 ist aus zwei Halbschalen aus Glas gebildet, zwischen denen das Leuchtmittel aufgenommen ist, in den gezeigten Ausführungsformen also die auf dem LED-Träger 11 angeordneten LED 12. Andere Materialien für den Leuchtkörper 2 sind möglich, sofern diese als lichtdurchlässige Materialien gebildet sind. Auch silikonbasierte Materialien und Materialien aus Kunststoff kommen in Betracht, die insbesondere als biokompatible Materialien gebildet sind. Auf dem Leuchtkörper 2 kann zur partiellen Ausleuchtung eine Maskierung als lokal begrenzte Abschirmung angeordnet sein.

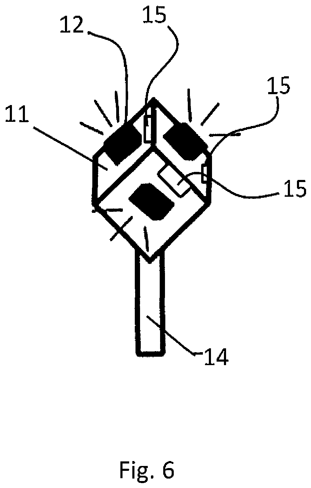

Figur 6 zeigt eine Ausführungsform, bei der im Leuchtkörper 2 auf dem LED-Träger 11 mindestens 1, bei dem gezeigten Beispiel 4 Photosensoren 15 vorhanden sind, deren Signal über eine der Versorgungsleitung 4 zugeordnete Signalleitung zu einer in der Steuereinheit 3 angeordneten Auswerteeinheit zum Schalten des Leuchtkörpers 2 zuführbar ist. Diese Ausführungsform kann als theragnostisches Implantatsystem genutzt werden, um nach entsprechender Gabe eines sich in Tumorzellen anreichernden fluoreszierenden Medikament und dem Auftreten von Fluoreszenz als Hinweis auf das Vorliegen von Tumorzellen die therapeutische Nutzung des Implantatsystems zu starten.

Mit einem derartigen Implantatsystem 1 besteht die Möglichkeit, dass nach der operativen Entfernung eines Tumors, insbesondere eines Hirntumors oder Glioblastoms, in die so entstandene Resektionshöhle der Leuchtkörper 2 eingesetzt wird, wobei die Versorgungsleitung 4 aus dem Körper, insbesondere der Schädelkalotte herausgeleitet und zur Zugentlastung mittels der Leitungsfahne 17 an der Schädelkalotte durch ein durch die Öffnung 18 hindurchreichendes Befestigungsmittel fixiert wird. Die Versorgungsleitung wird subkutan mittels eines Trokars in den Oberkörper des Patienten 13 verlegt. Dort wird dann die Versorgungsleitung 4 über die Koppelglieder 9, 10 mit dem Gehäuse 5 der Steuereinheit 3 verbunden, die über die Ösen 16 mit den umliegenden Weichteilen des Körpers verbunden wird, um ein Wandern im Körper zu verhindern. Für die Versorgungsleitung 4 wirkt dies auch als Zugentlastung am anderen, der Steuereinheit 3 zugewandten Ende.

Um die bei der Operation gebildete Resektionshöhle vollständig bis zur Resektionswand ausfüllen zu können, sind mehrere Leuchtkörper 2 unterschiedlichen Volumens bereitgestellt, so dass ein Leuchtkörper 2 geeigneter Größe ausgewählt werden kann. Denkbar ist auch, Leuchtkörper 2 mit Leuchtmitteln unterschiedlicher Abstrahlfrequenz vorzusehen, um je nach verwendetem Medikament dessen Photoaktivierung optimal zu ermöglichen.

Ergänzend besteht die Möglichkeit, dass die Steuereinheit 3 ein Reservoir für ein Medikament aufweist, dass das Reservoir über eine Medikamentenleitung mit dem Leuchtkörper 2 verbunden wird , der zur Abgabe des Medikamentes an das umliegende Gewebe eingerichtet ist und dass die Medikamentenleitung parallel zur Versorgungsleitung 4 verläuft, und dass der Leuchtkörper 2 Poren und/oder auf seiner Oberfläche Kanäle zur Verteilung der Medikamente aufweist und dass das Reservoir einen Medikamentenaufnahmepfad zur Befüllung mittels einer extrakorporalen Injektionsnadel aufweist. Auch kann das Implantatsystem 1 so ergänzt werden, dass dem Leuchtkörper 2 eine auf das Tumorgebiet gerichtete Kamera zugeordnet ist, deren Daten über eine zur Versorgungsleitung 4 parallelen Kameraleitung an die Steuereinheit 3 übermittelbar und/oder dort speicherbar und/oder kabellos an einen extrakorporalen Empfänger übertragbar sind.

Das entsprechend modular aufgebaute Implantatsystem 1 wird dann patientenspezifisch zusammengestellt durch Auswahl des geeigneten Leuchtkörpers 2 mit zugeordneter Versorgungsleitung und der Steuereinheit 3. Da eine Implantation und das dauerhafte Verbleiben im Körper des Patienten 13 beabsichtigt ist, sind der Leuchtkörper 2 und/oder die Versorgungsleitung 4 und/oder die Steuereinheit 3 mit einem biokompatiblen Material, beispielsweise medizinischem Silikon ummantelt.

### Bezugszeichenliste

- 1: Implantatsystem
- 2: Leuchtkörper
- 3: Steuereinheit
- 4: Versorgungsleitung
- 5: Gehäuse
- 6: Speicherzelle
- 7: Microcontroller
- 8: Steuerplatine
- 9: Koppelglied
- 10: Koppelglied
- 11: LED-Träger
- 12: LED
- 13: Patient
- 14: Anschlussglied
- 15: Photosensor
- 16: Öse
- 17: Leitungsfahne
- 18: Öffnung

## Patentansprüche

1. Implantatsystem für die photodynamische Therapie mit einem in eine Resektionshöhle implantierbaren Leuchtkörper (2) zur Abstrahlung von Licht und mit einer autonomen Steuereinheit (3), die über eine Versorgungsleitung (4) mit dem Leuchtkörper (2) verbindbar ist und ein Reservoir für mindestens ein Medikament aufweist, **dadurch gekennzeichnet, dass** das Reservoir über eine Medikamentenleitung mit dem Leuchtkörper (2) verbunden ist, der zur Abgabe des mindestens einen Medikamentes an das umliegende Gewebe eingerichtet ist, dass die Medikamentenleitung parallel zur Versorgungsleitung (4) verläuft, und dass der Leuchtkörper (2) Poren und/oder auf seiner Oberfläche Kanäle zur Verteilung der Medikamente aufweist.

2. Implantatsystem nach Anspruch 1, **dadurch gekennzeichnet, dass** der Leuchtkörper (2) aus einem lichtdurchlässigen Material gebildet ist.

3. Implantatsystem nach Anspruch 2, **dadurch gekennzeichnet, dass** im lichtdurchlässigen Material Streuzentren zur Förderung einer gleichmäßigen sphärischen Ausleuchtung ausgebildet sind.

4. Implantatsystem nach einem der Ansprüche 1 bis 3, **dadurch gekennzeichnet, dass** auf dem Leuchtkörper (2) zur partiellen Ausleuchtung eine Maskierung vorgesehen ist.

5. Implantatsystem nach einem der Ansprüche 1 bis 4, **dadurch gekennzeichnet, dass** in der Steuereinheit (3) eine Spannungsquelle angeordnet ist, und dass der Leuchtkörper (2) aus zwei Halbschalen gebildet ist, zwischen denen mindestens ein Leuchtmittel aufgenommen ist, das mittels der Versorgungsleitung (4) mit der Spannungsquelle verbindbar ist.

6. Implantatsystem nach Anspruch 4, **dadurch gekennzeichnet, dass** das Leuchtmittel mehrfach vorgesehen und dass jedes der Leuchtmittel durch eine LED (12) gebildet ist.

7. Implantatsystem nach Anspruch 6, **dadurch gekennzeichnet, dass** die Anordnung der LEDs (12) für eine gleichmäßige sphärische Ausleuchtung gewählt ist.

8. Implantatsystem nach Anspruch 6 oder 7, **dadurch gekennzeichnet, dass** die LEDs (12) auf einem LED-Träger (11) angeordnet sind, der als Anschlußglied (14) der LEDs (12) mit der Versorgungsleitung (4) ausgebildet ist.

9. Implantatsystem nach Anspruch 8, **dadurch gekennzeichnet, dass** der LED-Träger (11) die Form einer Kugel oder die Form eines Quaders, einschließlich der Sonderform eines Würfels, oder die Form einer Pyramide aufweist, auf deren Flächen im Flächenschwerpunkt die LEDs (12) angeordnet sind.

10. Implantatsystem nach Ansprüche 1 bis 9, **dadurch gekennzeichnet, dass** das Reservoir einen Medikamentenaufnahmepfad zur Befüllung mittels einer extrakorporalen Injektionsnadel aufweist.

11. Implantatsystem nach einem der Ansprüche 1 bis 10, **dadurch gekennzeichnet, dass** zwei Koppelglieder (9, 10) zur lösbaren Verbindung der Versorgungsleitung (4) mit der Steuereinheit (3) vorgesehen sind, von denen eines an der Außenwandung des Gehäuses (5) der Steuereinheit (3) angeordnet ist.

12. Implantatsystem nach Anspruch 11, **dadurch gekennzeichnet, dass** mehrere Leuchtkörper (2) unterschiedlichen Volumens vorgesehen sind zur wahlweisen Verbindung eines der Leuchtkörper (2) durch die zugeordnete Versorgungsleitung (4) mit dem Koppelglied (9) der Steuereinheit (3), und / oder dass mehrere Leuchtkörper (2) mit Leuchtmitteln unterschiedlicher Abstrahlfrequenz vorgesehen sind zur wahlweisen Verbindung eines der Leuchtkörper (2) durch die zugeordnete Versorgungsleitung (4) mit dem Koppelglied (9) der Steuereinheit (10).

13. Implantatsystem nach einem der Ansprüche 1 bis 12, **dadurch gekennzeichnet, dass** die Steuereinheit (3) über mindestens eine elektrische Speicherzelle (6) zur Spannungsversorgung der mindestens einen LED (12) sowie einer Steuerplatine (8) verfügt.

14. Implantatsystem nach einem der Ansprüche 1 bis 13, **dadurch gekennzeichnet, dass** im Inneren des Leuchtkörpers (2) mindestens ein Photosensor (15) angeordnet ist, dessen Signal über eine der Versorgungsleitung (4) zugeordnete Signalleitung zu einer in der Steuereinheit (3) angeordnete Auswerteeinheit zum Schalten des Leuchtkörpers (2) zuführbar ist.

15. Implantatsystem nach einem der Ansprüche 1 bis 14, **dadurch gekennzeichnet, dass** dem Leuchtkörper (2) eine auf das Tumorgebiet gerichtete Kamera mit einem beweglichen auswählbaren Bildbereich zugeordnet ist, deren Daten über eine zur Versorgungsleitung (4) parallelen Kameraleitung an die Steuereinheit (3) übermittelbar und/oder dort speicherbar und/oder kabellos an einen extrakorporalen Empfänger übertragbar sind.

## Claims

1. Implant system for photodynamic therapy with a luminous body (2) which can be implanted in a resection cavity for emitting light and with an autonomous control unit (3) which can be connected to the luminous body (2) via a supply line (4) and has a reservoir for at least one medicament, **characterised in that** the reservoir is connected to the luminous body (2) via a medicament line, which luminous body (2) is set up to deliver the at least one medicament to the surrounding tissue, **in that** the medicament line runs parallel to the supply line (4), and **in that** the luminous body (2) has pores and/or, on its surface, channels for distributing the medicaments.

2. Implant system according to claim 1, **characterized in that** the luminous body (2) is formed of a translucent material.

3. Implant system according to claim 2, **characterized in that** scattering centers are formed in the translucent material to promote uniform spherical illumination.

4. Implant system according to one of claims 1 to 3, **characterized in that** masking is provided on the luminous body (2) for partial illumination.

5. Implant system according to one of the claims 1 to 4, **characterized in that** a voltage source is arranged in the control unit (3), and **in that** the luminous body (2) is formed from two half shells, between which at least one luminous means is accommodated, which can be connected to the voltage source by means of the supply line (4).

6. An implant system according to claim 4, **characterized in that** said illuminating means is provided multiple times and that each of said illuminating means is formed by an LED (12).

7. Implant system according to claim 6, **characterized in that** the arrangement of LEDs (12) is selected for uniform spherical illumination.

8. Implant system according to claim 6 or 7, **characterized in that** the LEDs (12) are arranged on an LED carrier (11) which is formed as a connecting member (14) of the LEDs (12) with the supply line (4).

9. Implant system according to claim 8, **characterized in that** the LED carrier (11) has the shape of a sphere or the shape of a cuboid, including the special shape of a cube, or the shape of a pyramid, on the surfaces of which the LEDs (12) are arranged at the surface center of gravity.

10. Implant system according to claims 1 to 9, **characterized in that** the reservoir has a drug receiving path for filling by means of an extracorporeal injection needle.

11. Implant system according to any of claims 1 to 10, **characterized in that** two coupling members (9,10) are provided for releasably connecting the supply line (4) to the control unit (3), one of which is arranged on the outer wall of the housing (5) of the control unit (3).

12. Implant system according to claim 11, **characterized in that** a plurality of luminous bodies (2) of different volume are provided for selective connection of one of the luminous bodies (2) through the associated supply line (4) to the coupling member (9) of the control unit (3), and/or **in that** a plurality of luminous bodies (2) with luminous means of different radiation frequency are provided for selective connection of one of the luminous bodies (2) through the associated supply line (4) to the coupling member (9) of the control unit (10).

13. Implant system according to one of the claims 1 to 12, **characterized in that** the control unit (3) has at least one electrical storage cell (6) for supplying voltage to the at least one LED (12) and a control board (8).

14. Implant system according to one of claims 1 to 13, **characterized in that** at least one photosensor (15) is arranged in the interior of the luminous body (2), the signal of which photosensor (15) is fed via a signal line assigned to the supply line (4) to an evaluation unit arranged in the control unit for switching the luminous body (2).

15. Implant system according to one of the claims 1 to 14, **characterized in that** the luminous body (2) is assigned a camera which is directed at the tumor area and has a movable selectable image area, the data of which camera can be transmitted to the control unit (3) via a camera line parallel to the supply line (4) and/or can be stored there and/or can be transmitted wirelessly to an extracorporeal receiver.

## Revendications

1. Système d'implant pour la thérapie photodynamique avec un corps lumineux (2) implantable dans une cavité de résection pour l'émission de lumière et avec une unité de commande autonome (3) qui peut être reliée à la cavité de résection par une ligne de commande (4), d'alimentation (4) pouvant être reliée au corps lumineux (2) et un réservoir pour au moins un médicament, **caractérisé en ce que** le réservoir est relié par une conduite de médicament au corps lumineux (2), qui est destiné à délivrer le au moins un médicament au tissu environnant, **en ce que** la conduite de médicament est parallèle à la conduite d'alimentation (4), et **en ce que** le corps lumineux (2) présente des pores et/ou des canaux sur sa surface pour la distribution des médicaments.

2. Système d'implant selon la revendication 1, **caractérisé en ce que** le corps de l'implant est constitué d'une matière plastique, le corps lumineux (2) est formé d'un matériau translucide.

3. Système d'implant selon la revendication 2, **caractérisé en ce que** des centres de diffusion sont formés dans le matériau translucide pour favoriser une illumination sphérique uniforme.

4. Système d'implants selon l'une des revendications 1 à 3, **caractérisé en ce que** sur le corps lumineux (2) un masquage est prévu pour l'éclairage partiel.

5. Système d'implant selon l'une des revendications 1 à 4, **caractérisé en ce qu'**une source de tension est disposée dans l'unité de commande (3) et **en ce que** le corps lumineux (2) est constitué de deux demi-coques, entre lesquelles est logé au moins un moyen d'éclairage qui peut être relié à la source de tension au moyen de la ligne d'alimentation (4).

6. Système d'implant selon la revendication 4, **caractérisé en ce que** le moyen d'éclairage est prévu plusieurs fois et que chacun des moyens d'éclairage est formé par une LED (12).

7. Système d'implant selon la revendication 6, **caractérisé en ce que** la disposition des LED (12) est choisie pour obtenir un éclairage sphérique uniforme.

8. Système d'implant selon la revendication 6 ou 7, **caractérisé en ce que** les LED (12) sont disposées sur un support de LED (11) qui est conçu comme un élément de raccordement (14) des LED (12) avec la ligne d'alimentation (4).

9. Système d'implant selon la revendication 8, **caractérisé en ce que** le support de LED (11) est a la forme d'une sphère ou la forme d'un parallélépipède, y compris la forme spéciale d'un cube, ou la forme d'une pyramide, sur les faces de laquelle sont disposées les LED (12) au centre de gravité de la surface.

10. Système d'implant selon les revendications 1 à 9, **caractérisé en ce que** le réservoir comporte un chemin de réception de médicament pour le remplissage au moyen d'une aiguille d'injection extracorporelle.

11. Système d'implant selon l'une des revendications 1 à 10, **caractérisé en ce que** deux éléments de couplage (9, 10) sont prévus pour assurer une liaison amovible entre la conduite d'alimentation (4) avec l'unité de commande (3), dont l'un est disposé sur la paroi extérieure du boîtier (5) de l'unité de commande (3).

12. Système d'implant selon la revendication 11, **caractérisé en ce que** plusieurs corps lumineux (2) de volumes différents sont prévus pour relier au choix l'un des corps lumineux (2) par la ligne d'alimentation (4) associée à l'élément de couplage (9) de l'unité de commande (3), et / ou que plusieurs corps lumineux (2) sont prévus avec des moyens d'éclairage de fréquences de rayonnement différentes pour connexion sélective de l'un des corps lumineux (2) par le biais de la ligne d'alimentation (4) associée à l'élément de couplage (9) de l'unité de commande (10).

13. Système d'implant selon l'une quelconque des revendications 1 à 12, **caractérisé en ce que** l'unité de commande (3) dispose d'au moins une unité d'alimentation électrique cellule de mémoire électrique (6) pour l'alimentation en tension de la au moins une LED (12) ainsi qu'une carte de commande (8).

14. Système d'implant selon l'une des revendications 1 à 13, **caractérisé en ce qu'**au moins un photocapteur (15) est disposé à l'intérieur du corps lumineux (2), dont le signal est transmis par l'intermédiaire d'une ligne de transmission (4) reliée à la ligne de commande à une unité d'évaluation située dans l'unité de commande (3) pour commuter le corps lumineux (2).

15. Système d'implant selon l'une des revendications 1 à 14, **caractérisé en ce que** le corps lumineux (2) est équipé d'une caméra orientée vers la zone tumorale avec une zone d'image mobile sélectionnable dont les données peuvent être transmises à l'unité de commande (3) par l'intermédiaire d'une ligne de caméra parallèle à la ligne d'alimentation (4) et/ou être transmises sans fil à un récepteur extracorporel.
